(19) Europäisches Patentamt
European Patent Office
Office européen des brevets

(11) Numéro de publication : 0 283 410 B1

# (12) FASCICULE DE BREVET EUROPEEN

(45) Date de publication du fascicule du brevet : **26.06.91 Bulletin 91/26**

(51) Int. Cl.$^5$ : **C07C 37/62**, C07C 39/28, C07C 39/30

(21) Numéro de dépôt : **88420077.5**

(22) Date de dépôt : **03.03.88**

(54) **Procédé de chloration de l'orthochlorophénol.**

(30) Priorité : **05.03.87 FR 8703207**

(43) Date de publication de la demande : **21.09.88 Bulletin 88/38**

(45) Mention de la délivrance du brevet : **26.06.91 Bulletin 91/26**

(84) Etats contractants désignés : **AT BE CH DE ES GB GR IT LI LU NL SE**

(56) Documents cités :
**DE-A- 1 046 061**
**FR-A- 2 587 332**
**GB-A- 573 477**

(73) Titulaire : **RHONE-POULENC CHIMIE**
**25, quai Paul Doumer**
**F-92408 Courbevoie Cédex (FR)**

(72) Inventeur : **Leblanc, Jean-Claude**
**1 Boulevard Joffre**
**F-38000 Grenoble (FR)**
Inventeur : **Ratton, Serge**
**Hameau de Belmont Vaulx-Milieu**
**F-38090 Villefontaine (FR)**
Inventeur : **Besson, Bernard**
**15, rue de Moucherotte**
**F-38800 Pont de Claix (FR)**
Inventeur : **Desmurs, Jean-Roger**
**La Jonquière Route de Ternay Communay**
**St-Symphorien d'Ozon (FR)**

(74) Mandataire : **Vignally, Noel et al**
**Rhône-Poulenc Interservices Service Brevets Chimie Centre de Recherches des Carrières**
**B.P. 62**
**F-69192 Saint-Fons Cédex (FR)**

**EP 0 283 410 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne la chloration de l'orthochlorophénol en dichloro-2,6 phénol.

Le brevet anglais 537 477 a décrit un procédé de chloration des crésols en monochlorocrésols, et plus spécifiquement la réaction de l'ortho-crésol fondu avec le chlore, en l'absence de tout solvant. Le procédé n'est pas sélectif et conduit à un mélange de chloro-6 ortho-crésol et chloro-4 ortho-crésol, avec prédominance de ce dernier.

La demande de brevet allemand No. 3.318.791 a décrit un procédé de chloration sélective du phénol en orthochlorophénol, consistant à opérer dans un milieu solvant non polaire perchloré et en présence d'une amine à chaîne ramifiée.

La demande de brevet français No. 85/03802 a décrit la chloration sélective de divers composés, dont l'orthochlorophénol, en position ortho par rapport à la fonction phénolique, en milieu solvant aprotique apolaire et en présence d'une amine primaire, secondaire ou tertiaire.

Ce procédé est très sélectif, mais il implique d'utiliser un solvant, ce qui complique le traitement ultérieur.

Il a maintenant été trouvé que l'on peut améliorer la sélectivité de la chloration de l'orthochlorophénol en dichloro-2,6 phénol, par le chlore gazeux, sans adjonction d'un tiers solvant.

Plus précisément, il s'agit d'un procédé de chloration par le chlore gazeux d'orthochlorophénol en dichloro-2,6 phénol, caractérisé en ce que l'on opère à l'état fondu et en présence d'une quantité efficace d'au moins une amine primaire, secondaire ou tertiaire.

Par amine on entend dans le présent texte tout composé, liquide ou solide dans les conditions opératoires du procédé, comportant une ou plusieurs fonctions amine.

Un tel composé peut également comporter une ou plusieurs autres fonctions chimiques, telles que par exemple la fonction hydroxyle, la fonction acide carboxylique, la fonction ester d'acide carboxylique, la fonction amide ou la fonction imine.

Il est bien évident que les amines utilisées peuvent être introduites également sous la forme de leurs sels et plus particulièrement de leurs chlorhydrates respectifs.

Dans le présent texte, le terme "amine" englobe également l'ammoniac ainsi que les sels et notamment les chlorhydrates d'amines.

Les amines utilisées comme catalyseur dans le présent procédé sont plus particulièrement les amines de formule générale (I) :

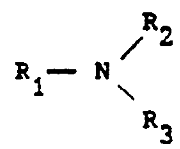

(I)

dans laquelle :

– $R_1$, $R_2$ et $R_3$, identiques ou différents, représentent :

- un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther –O– ou fonctions hydroxyle, amine, acide carboxylique, ester d'acide carboxylique, amide ou imine ;
- un radical phényle, un radical cyclohexyle, un radical cycloheptyle ou un radical cyclopentyle ;
- un radical phénylalkyle, cyclohexylalkyle, cycloheptylalkyle ou cyclopentylalkyle dont la partie alkyle comporte 1 à 4 atomes de carbone ;
- un atome d'hydrogène ;

– $R_1$ peut représenter un groupement $NH_2$ ;

– $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;

– $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;

– $R_1$, $R_2$ et $R_3$ forment ensemble et avec l'atome d'azote un hétérocycle insaturé éventuellement substitué par un ou deux groupements méthyle ou éthyle ;

– $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

A titre d'exemples illustratifs, on peut citer comme amines de formule (I) :

– l'ammoniac ;

– des amines primaires telles que la n-proplyamine, l'isopropylamine, l'isobutylamine, la n-butylamine, la tertiobutylamine, la n-pentylamine, la méthyl-2 butylamine, la méthyl-3 butylamine, la n-hexylamine, l'éthyl-2 hexylamine, l'aniline, la laurylamine, la cyclohexylamine, la cyclopentylamine, la benzylamine, la guanidine, l'acétamidine, l'ester éthylique de la glycine, l'éthanolamine, l'éthylènediamine, l'hexaméthylènediamine, la N-aminoéthylpyrrolidine, la pyrazoline, la lysine, la N-aminomorpholine, la N-aminopipéridine ;

– des amines secondaires telles que la dibutylamine, la dipropylamine, la méthylpropylamine, la méthylbutylamine, la méthylisobutylamine, la méthyltertiobutylamine, la méthylbenzylamine, la ditertiobutylamine, la méthyl-1 cyclopentylamine, la methyl-1 cyclohexylamine, la dicyclohexylamine, la morpholine, l'imidazole, la pyrrolidine, l'imidazolidine, la pipérazine, l'indole ;

– des amines tertiaires telles que la triéthylamine, la tributylamine, la pyridine, la tris(dioxa-3,6 heptyl) amine, le diaza-1,8 bicyclo(5,4,0)undécène-7.

On peut utiliser également des composés aminés comme l'hydrazine ou ses dérivés, notamment les dérivés obtenus par substitution d'un ou de deux atomes d'hydrogène par des radicaux alkyles, aryles, cycloaliphatiques ou hétérocycliques.

La quantité d'amine utilisée dans le procédé peut varier dans de très larges limites.

Habituellement elle représente en poids par rapport au poids du milieu réactionnel de 0,005% à 10%. Préférentiellement on mettra en oeuvre de 0,015% à 5% en poids d'amine par rapport au milieu réactionnel, afin d'avoir une efficacité suffisante, tout en n'ayant pas une quantité excessive d'amine.

Parmi les amines de formule générale (I), on préfère dans le cadre du présent procédé, les amines primaires ou secondaires de formule (II) :

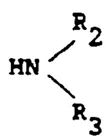
(II)

dans laquelle :

– $R_2$ ou $R_3$ représente un atome d'hydrogène ;

– $R_2$ et $R_3$, identiques ou différents, représentent :

- un radical alkyle linéaire ayant 1 à 10 atomes de carbone ;
- un radical alkyle secondaire ayant 3 à 10 atomes de carbone ;
- un radical alkyle tertiaire ayant 4 à 10 atomes de carbone ;
- un radical cyclohexyle ou cyclopentyle ;
- un radical phényle ;
- un radical benzyle ou phénéthyle ;

– $R_2$ et $R_3$ forment ensemble, avec l'atome d'azote et avec un autre atome d'azote et/ou d'oxygène, un hétérocycle saturé ou comportant une ou plusieurs insaturations ;

– $R_2$ et/ou $R_3$ comportent une ou plusieurs fonctions amine, hydroxyle ou ester d'acide carboxylique.

A titre d'exemples d'amines primaires de formule générale (II), on peut citer la n-propylamine, l'isopropylamine, la n-butylamine, l'isobutylamine, la tertiobutylamine, la n-pentylamine, la méthyl-2 pentylamine, la méthyl-3 pentylamine, l'éthyl-2 hexylamine, la laurylamine, la cyclohexylamine, la cyclopentylamine, la benzylamine, l'ester éthylique de la glycine et l'éthanolamine.

En ce qui concerne plus particulièrement les amines secondaires de formule générale (II), on préfère encore plus spécialement celles pour lesquelles au moins un des symboles $R_2$ et $R_3$ et de préférence les deux symboles $R_2$ et $R_3$ représentent :

- un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;
- un radical cyclohexyle ou cyclopentyle ;

et celles pour lesquelles $R_2$ et $R_3$ forment avec l'atome d'azote un hétérocycle comportant éventuellement un autre atome d'azote ou un atome d'oxygène.

A titre d'exemples de telles amines secondaires, on peut citer la diisopropylamine, la diisobutylamine, la

dicyclohexylamine, la morpholine, l'imidazole.

Une variante très intéressante du procédé de l'invention consiste à opérer la chloration de l'orthochlorophénol dilué dans au moins un polychlorophénol.

Plus précisément cette variante préférée consiste donc dans un procédé de chloration par le chlore gazeux d'orthochlorophénol en dichloro-2,6 phénol, caractérisé en ce que l'on opère à l'état fondu en présence :

– d'au moins un polychlorophénol en quantité telle que le rapport pondéral orthochlorophénol/ensemble des chlorophénols soit inférieur ou égal à 30%,

– et en présence d'une quantité efficace d'au moins une amine primaire, secondaire ou tertiaire.

Habituellement, comme dans le procédé général de chloration, l'amine représente en poids par rapport au poids du milieu réactionnel de 0,005% à 10%.

De préférence, comme cela a été indiqué précédemment, on utilise de 0,015% à 5% en poids d'amine par rapport au poids du milieu réactionnel.

Les amines (ou leurs chlorhydrates) sont celles qui ont été définies dans le cadre général du procédé de l'invention.

Les polychlorophénols, en présence desquels cette variante du procédé de l'invention est mise en oeuvre, sont essentiellement le dichloro-2,4 phénol, le dichloro-2,6 phénol, le trichloro-2,4,6 phénol. Il peut y avoir également dans le mélange réactionnel d'autres polychlorophénols tels que le tétrachloro-2,3,4,6 ou le pentachlorophénol.

La chloration par le chlore gazeux de l'orthochlorophénol seul à l'état fondu conduit à une majorité de dichloro-2,6 phénol.

La variante préférée de chloration de l'orthochlorophénol à l'état fondu, dilué dans les polychlorophénols, procure une sélectivité encore plus importante de la chloration en dichloro-2,6 phénol par rapport à la chloration en dichloro-2,4 phénol.

Généralement, on peut obtenir par cette variante du procédé de l'invention une sélectivité égale ou supérieure à 0,70 mole de dichloro-2,6 phénol formé pour 1 mole d'orthochlorophénol engagé.

Le dichloro-2,4 phénol qui se forme en plus faible proportion est transformé pour sa plus grande part en trichloro-2,4,6 phénol.

La température à laquelle est mis en oeuvre le procédé selon l'invention est généralement comprise entre le point de fusion de l'orthochlorophénol et des polychlorophénols éventuellement présents et 150°C.

En pratique cette température varie de 65°C à 120°C, mais sans que ces valeurs soient critiques.

Le débit de chlore injecté dépend à la fois de l'appareillage et de la concentration en amine et en orthochlorophénol dans le milieu réactionnel.

En règle générale, plus la concentration en amine sera élevée, en particulier par rapport à l'orthochlorophénol présent, plus le débit pourra être important. De même une concentration en orthochlorophénol plus élevée dans le milieu autorise un débit plus important de chlore.

La présence d'amine permet une bonne fixation du chlore. Aussi n'est-il pas nécessaire généralement d'introduire un excès de chlore par rapport à la quantité stoechiométrique.

Les problèmes de recyclage de l'excès de chlore ou de traitement des effluents gazeux deviennent ainsi moins difficiles à résoudre.

Le chlore peut être utilisé seul ou être dilué par un gaz inerte, tel que l'azote par exemple. La présence d'un gaz inerte permet, si nécessaire, d'augmenter le débit gazeux sans augmenter la quantité de chlore introduite en un temps donné.

Le procédé de l'invention peut être réalisé de manière continue ou discontinue.

Il est très intéressant d'appliquer le présent procédé à un mélange d'orthochlorophénol et de trichloro-2,4,6 phénol. La majeure partie de l'orthochlorophénol est transformée en dichloro-2,6 phénol tandis qu'une autre partie est transformée en dichloro-2,4 phénol qui est lui-même chloré en trichloro-2,4,6 phénol. En fin de chloration, on obtient donc un mélange de dichloro-2,6 phénol et de trichloro-2,4,6 phénol, qui sont deux produits ayant des débouchés, notamment comme intermédiaires pour la synthèse de produits phytosanitaires.

Une autre variante du présent procédé consiste à chlorer tout d'abord de l'orthochlorophénol à l'état fondu en présence d'une amine telle que définie précédemment. Cette chloration donne une majorité de dichloro-2,6 phénol ainsi que du dichloro-2,4 phénol et du trichloro-2,4,6 phénol.

Lorsque la concentration en orthochlorophénol dans le milieu atteint une valeur inférieure ou égale à environ 30% en poids, on injecte de l'orthochlorophénol en continu de manière concomitante avec l'injection du chlore gazeux.

Les débits sont tels que la concentration pondérale en orthochlorophénol dans le milieu réactionnel soit en permanence inférieure ou égale à 30%. Ainsi l'orthochlorophénol injecté est transformé très majoritairement en dichloro-2,6 phénol.

Lorsqu'on le souhaite, on arrête l'addition d'orthochlorophénol et on poursuit la chloration jusqu'à disparition complète de l'orthochlorophénol présent dans le milieu réactionnel.

En fin de réaction, on a ainsi un mélange de dichloro-2,6 phénol et de trichloro-2,4,6 phénol.

Ce mélange sera d'autant plus riche en dichloro-2,6 phénol que l'on aura injecté une plus grande quantité d'orthochlorophénol au cours du deuxième stade de la chloration.

Enfin une autre variante du procédé selon l'invention consiste dans la préparation de trichloro-2,4,6 phénol, à partir du dichloro-2,6 phénol ou, le cas échéant, à partir du mélange de dichloro-2,6 phénol et de trichloro-2,4,6 phénol obtenu dans le cadre du procédé.

Cette variante consiste à introduire dans le milieu réactionnel contenant le dichloro-2,6 phénol, éventuellement en mélange avec du trichloro-2,4,6 phénol, une quantité efficace d'un acide fort ou d'un acide de Lewis.

On entend par acide fort un acide protonique ayant une fonction d'acidité Ho inférieure ou égale à −5.

Comme exemples non limitatifs de tels acides forts, on peut citer l'acide sulfurique, l'acide perchlorique, l'acide trifluorométhanesulfonique, l'acide chlorosulfonique, l'acide fluorosulfonique, l'acide pyrosulfurique et des résines acides comportant des groupements fluorosulfoniques.

Par acide de Lewis, on entend selon la définition usuelle, des composés accepteurs de doublets électroniques. On peut utiliser notamment les acides de Lewis cités dans l'ouvrage édité par G. A. OLAH "Friedel-Crafts and Related Reactions" tome I, pages 191 à 197 (1963).

Les acides de Lewis pouvant servir dans la variante du procédé, sont plus particulièrement les halogénures d'éléments des groupes 3a, 4a, 5a, 1b, 2b, 4b, 5b, 6b, 7b et 8 de la classification périodique des éléments, qui sont liquides ou solides dans les conditions opératoires, tels que les chlorures, bromures, fluorures et iodures d'aluminium, d'étain, de phosphore, d'antimoine, d'arsenic, de bismuth, de titane, de tantale, de nobium, de zirconium, de vanadium, de tungstène, de molybdène, de fer, de cobalt, de nickel, de cuivre, de zinc et de cadmium.

Comme exemples spécifiques de tels halogénures, on peut citer le chlorure d'aluminium, le bromure d'aluminium, les chlorures stannique et stanneux, les bromures stannique et stanneux, le trichlorure de bismuth, le tétrachlorure de titane, le tétrachlorure de zirconium, le pentafluorure d'antimoine, l'hexachlorure de tungstène, les chlorures de molybdène, le chlorure ferrique, le chlorure ferreux, le bromure ferrique, le bromure ferreux, le chlorure cuivreux, le chlorure cuivrique et le chlorure de zinc.

Parmi ces acides de Lewis, on préfère le chlorure d'aluminium, le chlorure ferrique, le tétrachlorure de zirconium et le tétrachlorure de titane.

On peut également utiliser des complexes de certains acides de Lewis avec un hydracide, dans la mesure où ces complexes sont liquides ou solides dans les conditions de la réaction. Ainsi on peut citer par exemple le complexe $SbF_5$, HF.

Généralement la quantité d'acide fort ou d'acide de Lewis utilisée est telle que le rapport pondéral acide fort/dichloro-2,6 phénol ou acide de Lewis/dichloro-2,6 phénol est de 0,01% à 10%.

De préférence, ces rapports pondéraux sont de 0,1% à 5%.

Comme dans le procédé les réactifs sont à l'état fondu, on préfère plus particulièrement utiliser un acide protonique fort.

Ainsi on obtient avec excellent rendement du trichloro-2,4,6 phénol contenant généralement moins de 3% en poids de pentachloro-2,4,5,6,6 cyclohexène-2 one.

Les exemples qui suivent illustrent différentes variantes de la présente invention.

EXEMPLE 1

Dans un réacteur en verre de 500 cm3, équipé d'un tube plongeant muni d'une extrémité en verre fritté, d'une gaine thermométrique et d'un réfrigérant ascendant à eau, on charge :

- 35,5 g d'orthochlorophénol (0,276 mole) : 14,2 % en moles
- 330 g de trichloro-2,4,6 phénol (1,662mole) : 85,8 % en moles
- 0,4 g de dibutylamine (environ 0,1 % en poids par rapport au mélange réactionnel).

On chauffe à 70°C environ à l'aide d'un bain thermostaté, avant d'envoyer le chlore gazeux par le tube plongeant, avec un débit de 20 litres/heure. On maintient la température entre 65°C et 75°C pendant la chloration.

Après 6 minutes de chloration, on dose par chromatographie en phase gazeuse la composition molaire de la masse réactionnelle.

On trouve :

- orthochlorophénol : 6,45 %
- dichloro-2,6 phénol : 5,8 %
- dichloro-2,4 phénol : 1,75 %
- trichloro-2,4,6 phénol : 86,0 %

Cela montre que la sélectivité de la chloration de l'orthochlorophénol en dichloro-2,6 phénol est de 0,75 mole/mole.

La chloration est poursuivie pendant 12 minutes, jusqu'à disparition pratiquement totale de l'orthochlorophénol et du dichloro-2,4 phénol (moins de 0,2% dans le mélange).

On obtient le mélange suivant (% molaire déterminé par chromatographie en phase gazeuse) :

- dichloro-2,6 phénol : 10,4 %
- trichloro-2,4,6 phénol : 89,6 %

La sélectivité est de 0,73 mole de dichloro-2,6 phénol pour 1 mole d'orthochlorophénol engagé.

EXEMPLE 2

On répète l'exemple 1 avec les charges suivantes :

- 55 g d'orthochlorophénol (0,428 moles) : 27,8 % en moles,
- 220 g de trichloro-2,4,6 phénol (1,108 mole) : 72,2 % en moles,
- 0,28 g de diisopropylamine (0,1 % en poids par rapport au mélange réactionnel).

On chlore dans les conditions de l'exemple 1, de manière à transformer tout l'orthochlorophénol ainsi que le dichloro-2,4 phénol formé.

On obtient le mélange final suivant (% molaire déterminé par chromatographie en phase gazeuse) :

- dichloro-2,6 phénol : 20,9 %
- trichloro-2,4,6 phénol : 79,1 %

Cela correspond à une sélectivité de 0,75 mole de dichloro-2,6 phénol formé pour 1 mole d'orthochlorophénol engagé.

EXEMPLE 3

Dans l'appareillage décrit dans l'exemple 1, on charge :

- 240 g d'orthochlorophénol (1,712 mole)
- 2,4 g de dibutylamine (1,0 % en poids par rapport à l'orthochlorophénol)

On chauffe à 70°C, puis on envoie du chlore gazeux par le tube plongeant, avec un débit de 40 litres/heure environ, jusqu'à ce que la concentration molaire en orthochlorophénol soit inférieure à 10% (évolution suivie par chromatographie en phase gazeuse).

Ainsi après une chloration d'une heure environ, le mélange réactionnel a la composition molaire suivante:

- orthochlorophénol : 6,8 %
- dichloro-2,6 phénol : 50,9 %
- dichloro-2,4 phénol : 39,4 %
- trichloro-2,4,6 phénol : 2,9 %

La sélectivité de la réaction à ce stade est de 0,55 mole de dichloro-2,6 phénol formé pour une mole d'orthochlorophénol engagé.

Ce mélange est transféré dans un réacteur en verre de 1 litre, équipé comme le précédent, mais comportant en outre un tube d'injection pour l'orthochlorophénol.

En maintenant la température à 70-75°C, on injecte de manière concomitante dans le réacteur :

- 240 g/heure d'orthochlorophénol
- du chlore avec un débit de 45 litres/heure.

On contrôle, par dosages chromatographiques, que la concentration en orthochlorophénol demeure inférieure à 10% en poids dans le milieu (elle varie entre 3% et 8% en poids au cours de l'essai).

Après 8 heures de chloration, on obtient un mélange réactionnel ayant la composition molaire suivante :

- orthochlorophénol : 3,7 %
- dichloro-2,6 phénol : 65,0 %
- dichloro-2,4 phénol : 10,4 %
- trichloro-2,4,6 phénol : 20,9 %

L'addition d'orthochlorophénol est alors arrêtée et la chloration est poursuivie jusqu'à disparition complète de l'orthochlorophénol présent et du dichloro-2,4 phénol.

Le mélange réactionnel final a la composition molaire suivante :

- dichloro-2,6 phénol : 66,0 %
- trichloro-2,4,6 phénol : 34,0 %

## EXEMPLE 4

On répète l'exemple 3 en utilisant de la diisopropylamine comme catalyseur de chloration.

On charge tout d'abord :

- 235 g d'orthochlorophénol,
- 2,4 g de diisopropylamine.

On chauffe vers 70°C, puis on envoie le chlore gazeux par le tube plongeant, avec un débit de 40 litres/heure environ, pendant 1 h 20 min.

La composition molaire du mélange réactionnel est alors la suivante :

- orthochlorophénol : 0,5 %
- dichloro-2,6 phénol : 60,3 %
- dichloro-2,4 phénol : 38,1 %
- trichloro-2,4,6 phénol : 1,1 %

La sélectivité à ce stade est de 0,60 mole de dichloro-2,6 phénol formé pour 1 mole d'orthochlorophénol engagé.

On opère ensuite comme décrit dans l'exemple 5 : dans le deuxième réacteur, on injecte de manière concomitante pendant 8 heures :

- 240 g/heure d'orthochlorophénol,
- 45 litres/heure environ de chlore gazeux.

On contrôle, par dosages chromatographiques, que la concentration en orthochlorophénol demeure inférieure à 10% en poids dans le milieu.

Après 8 heures de chloration, le mélange réactionnel a la composition molaire suivante :

- orthochlorophénol : 2,7 %
- dichloro-2,6 phénol : 65,4 %
- dichloro-2,4 phénol : 8,4 %

– trichloro-2,4,6 phénol : 23,5 %

L'addition d'orthochlorophénol est alors arrêtée et la chloration est poursuivie jusqu'à disparition complète de l'orthochlorophénol présent et du dichloro-2,4 phénol.

Le mélange réactionnel final a la composition molaire suivante :

– dichloro-2,6 phénol : 70,0 %
– trichloro-2,4,6 phénol : 30,0 %

EXEMPLE 5

Dans un réacteur en verre de 500 $cm^3$, équipé comme indiqué dans l'exemple 1, on charge :

- 35,5 g d'orthochlorophénol (0,276 mole) : 14,2 % en moles
- 330 g de trichloro-2,4,6 phénol (1,662 mole) : 85,8 % en moles
- 0,4 g de dibutylamine (0,0031 mole) (0,11 % en poids par rapport au milieu réactionnel).

On chauffe à 70°C et on envoie le chlore gazeux par le tube plongeant, avec un débit de 15 litres/heure.

On maintient la température à environ 70°C et l'avancement de la réaction est suivi par analyses d'échantillons prélevés au cours du temps.

Après 14 minutes tout l'orthochlorophénol a été transformé.

La masse réactionnelle a la composition molaire suivante, déterminée par chromatographie en phase gazeuse :

– orthochlorophénol : < 0,1 %
– dichloro-2,6 phénol : 11,8 %
– dichloro-2,4 phénol : 1,3 %
– trichloro-2,4,6 phénol : 86,9 %

La sélectivité de la chloration de l'orthochlorophénol en dichloro-2,6 phénol est de 0,83 mole/mole.

ESSAI COMPARATIF A

On répète, à titre de comparaison, l'exemple 5, sans mettre d'amine.

Les conditions de chloration sont les mêmes que dans l'exemple 5, mais la durée nécessaire pour que tout l'orthochlorophénol soit transformé est de 17 minutes.

La composition molaire du mélange réactionnel final est la suivante :

– orthochlorophénol : < 0,1 %
– dichloro-2,6 phénol : 4,3 %
– dichloro-2,4 phénol : 8,9 %
– trichloro-2,4,6 phénol : 86,8 %

La sélectivité de la chloration de l'orthochlorophénol en dichloro-2,6 phénol est de 0,30 mole/mole.

EXEMPLE 6

On opère comme dans l'exemple 5, mais en diminuant le rapport pondéral amine/milieu réactionnel. On charge :

- 27,0 g d'orthochlorophénol (0,21 mole)
- 230 g de trichloro-2,4,6 phénol (1,165 mole)
- 0,130 g de dibutylamine (0,001 mole) : 0,05 % en poids d'amine/milieu réactionnel.

Les conditions de chloration sont identiques à celles de l'exemple 5.

Après 17 minutes tout l'orthochlorophénol a été transformé.

La composition molaire du mélange réactionnel final est la suivante :

| | |
|---|---|
| – orthochlorophénol : | < 0,1 % |
| – dichloro-2,6 phénol : | 11,2 % |
| – dichloro-2,4 phénol : | 3,0 % |
| – trichloro-2,4,6 phénol : | 85,8 % |

La sélectivité est de 0,73 mole de dichloro-2,6 phénol formé pour 1 mole d'orthochlorophénol engagé.

ESSAI COMPARATIF B

Dans un réacteur en verre de 100 cm3, équipé comme dans l'exemple 1, on charge 38,6 g (0,3 mole d'orthochlorophénol).

L'orthochlorophénol est chauffé à 50°C puis à cette température on introduit 0,27 mole de chlore avec un débit de 5 l/h.

A la fin de l'addition du chlore, le réacteur est purgé à l'azote. Le mélange réactionnel (48,14 g est analysé en chromatographie en phase gazeuse. La composition molaire est la suivante :

| | |
|---|---|
| – orthochlorophénol : | 11,5 % |
| – dichloro-2,6 phénol : | 20,8 % |
| – dichloro-2,4 phénol : | 66,3 % |
| – trichloro-2,4,6 phénol : | 1,5 % |

La sélectivité de la chloration de l'orthochlorophénol en dichloro-2,6 phénol est de 0,24 mole/mole.

EXEMPLE 7

On opère comme dans l'exemple 5, avec les charges suivantes :

- 55,0 g d'orthochlorophénol (0,428 mole)
- 220 g de trichloro-2,4,6 phénol (1,108 mole)
- 0,275 g de dibutylamine (0,0021 mole).

Les conditions de chloration sont les mêmes que dans l'exemple 5.

Après 34 minutes, tout l'orthochlorophénol a été transformé.

La composition molaire du mélange réactionnel final est la suivante :

| | |
|---|---|
| – orthochlorophénol : | 0 % |
| – dichloro-2,6 phénol : | 22,4 % |
| – dichloro-2,4 phénol : | 2,1 % |
| – trichloro-2,4,6 phénol : | 75,5 % |

La sélectivité est de 0,81 mole de dichloro-2,6 phénol formé pour 1 mole d'orthochlorophénol engagé.

EXEMPLE 8

On opère comme dans l'exemple 5, avec les charges suivantes :

- 100 g d'orthochlorophénol (0,778 mole)
- 150 g de trichloro-2,4,6 phénol (0,754 mole)
- 0,250 g de dibutylamine (0,0019 mole).

Le débit de chlore est de 30 litres/heure et la température est de 70°C environ.
Après 43 minutes, tout l'orthochlorophénol à été transformé.
La composition molaire du mélange réactionnel final est la suivante :

| | |
|---|---|
| – orthochlorophénol : | < 0,1 % |
| – dichloro-2,6 phénol : | 31,1 % |
| – dichloro-2,4 phénol : | 17,7 % |
| – trichloro-2,4,6 phénol : | 55,3 % |

La sélectivité est de 0,62 mole de dichloro-2,6 phénol pour 1 mole d'orthochlorophénol engagé.

EXEMPLE 9

On opère comme dans l'exemple 5, avec les charges suivantes :

- 240 g d'orthochlorophénol (1,867 mole)
- 7,0 g de dibutylamine (2,9 % en poids par rapport à l'orthochlorophénol).

Le débit de chlore est de 30 litres/heure et la température est de 70°C environ.
Après 71 minutes de réaction la composition molaire du mélange réactionnel est la suivante :

| | |
|---|---|
| – orthochlorophénol : | 1,9 % |
| – dichloro-2,6 phénol : | 49,3 % |
| – dichloro-2,4 phénol : | 44,2 % |
| – trichloro-2,4,6 phénol : | 4,6 % |

La sélectivité est de 0,51 mole de dichloro-2,6 phénol pour 1 mole d'orthochlorophénol engagé.

EXEMPLE 10

Dans un réacteur en verre de 200 cm3 équipé comme décrit dans l'exemple 1, on charge :

- orthochlorophénol : 9,0 g (70 mmol)
- dichloro-2,6 phénol : 51 g (312,9 mmol)
- diisopropylamine : 0,06 g

Le mélange réactionnel est chauffé à 70°C sous agitation et le chlore gazeux est introduit avec un débit de 5 l/heure pendant 18 min 50 s, ce qui représente une quantité de chlore introduite de 70 mmol.

On analyse par CPG et CLHP le mélange réactionnel. On obtient les résultats suivants :

- TT de l'orthochlorophénol : 94,0 %
- RT en dichloro-2,6 phénol : 64,3 %
- RT en dichloro-2,4 phénol : 22,5 %
- RT en trichloro-2,4,6 phénol : 6,7 %.

On introduit alors 0,3 g d'acide trifluorométhanesulfonique (0,5% en poids), puis on introduit de nouveau du chlore gazeux avec un débit de 5 l/h pendant 2 heures, ce qui représente une quantité introduite de chlore de 446 mmol.

Après purge à l'azote, on analyse par CPG et CLHP le mélange réactionnel final.

On obtient les résultats suivants :

- TT de l'orthochlorophénol et du dichloro-2,6 phénol : 98,8 %
- RT en trichloro-2,4,6 phénol par rapport à l'orthochlorophénol et au dichloro-2,6 phénol transformés : 97,5 %
- RT en pentachlorocyclohexènone : 2,7 %.

**Revendications**

1. Procédé de chloration par le chlore gazeux d'orthochlorophénol en dichloro-2,6 phénol, caractérisé en ce que l'on opère à l'état fondu et en présence d'une quantité efficace d'au moins une amine primaire, secondaire ou tertiaire.

2. Procédé selon la revendication 1 de chloration par le chlore gazeux, d'orthochlorophénol en dichloro-2,6 phénol, caractérisé en ce que l'on opère à l'état fondu en présence :

– d'au moins un polychlorophénol en quantité telle que le rapport pondéral orthochlorophénol/ensemble des chlorophénols soit inférieur ou égal à 30%,

– et en présence d'une quantité efficace d'au moins une amine primaire, secondaire ou tertiaire.

3. Procédé selon l'une des revendications 1 ou 2, caractérisé en ce que l'amine utilisée est une amine de formule générale (I) :

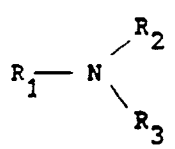

(I)

dans laquelle :

– $R_1$, $R_2$ et $R_3$, identiques ou différents, représentent :

• un radical alkyle linéaire ayant de 1 à 12 atomes de carbone, un radical alkyle secondaire ayant 3 à 12 atomes de carbone ou un radical alkyle tertiaire ayant 4 à 12 atomes de carbone, ces radicaux alkyles pouvant comporter une ou deux fonctions éther –O– ou fonctions hydroxyle, amine, acide carboxylique, ester d'acide carboxylique, amide ou imine ;

• un radical phényle, un radical cyclohexyle, un radical cycloheptyle ou un radical cyclopentyle ;

• un radical phénylalkyle, cyclohexylalkyle, cycloheptylalkyle ou cyclopentylalkyle dont la partie alkyle comporte 1 à 4 atomes de carbone ;

• un atome d'hydrogène ;

– $R_1$ peut représenter un groupement $NH_2$ ;

– $R_2$ et $R_3$ forment ensemble et avec l'atome d'azote un hétérocycle, saturé ou comportant une ou plusieurs doubles liaisons, éventuellement substitué par un ou plusieurs groupements alkyles ayant 1 à 4 atomes de carbone ;

– $R_2$ et $R_3$ ou $R_1$, $R_2$ et $R_3$ forment ensemble, avec l'atome d'azote et avec un ou plusieurs atomes d'azote et/ou d'oxygène et/ou de soufre, un hétérocycle, saturé ou insaturé, éventuellement substitué par un ou plusieurs groupements alkyles ayant 1 à 4 atomes de carbone ;

– $R_1$, $R_2$ et $R_3$ forment ensemble et avec l'atome d'azote un hétérocycle insaturé éventuellement substitué par un ou deux groupements méthyle ou éthyle ;

– $R_2$ et $R_3$ ou $R_1$, $R_2$ et $R_3$ forment ensemble, avec l'atome d'azote et éventuellement avec un ou plusieurs atomes d'azote et/ou d'oxygène et/ou de soufre, un composé polycyclique, saturé ou insaturé, éventuellement substitué par un ou plusieurs groupements alkyles ayant 1 à 4 atomes de carbone.

4. Procédé selon l'une des revendications 1 à 3, caractérisé en ce que la quantité d'amine utilisée représente en poids par rapport au poids du milieu réactionnel de 0,005% à 10%, et de préférence de 0,015% à 5%.

5. Procédé selon l'une des revendications 1 à 4, caractérisé en ce que l'on utilise des amines primaires ou secondaires de formule générale (II) :

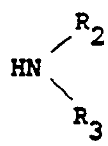 (II)

dans laquelle :

– $R_2$ ou $R_3$ représente un atome d'hydrogène ;

–$R_2$ et $R_3$, identiques ou différents, représentent :

• un radical alkyle linéaire ayant 1 à 10 atomes de carbone ;

• un radical alkyle secondaire ayant 3 à 10 atomes de carbone ;

• un radical alkyle tertiaire ayant 4 à 10 atomes de carbone ;

• un radical cyclohexyle ou cyclopentyle ;

• un radical phényle ; • un radical benzyle ou phénéthyle ;

– $R_2$ et $R_3$ forment ensemble, avec l'atome d'azote et avec un autre atome d'azote et/ou d'oxygène, un hétérocycle saturé ou comportant une ou plusieurs insaturations ;

– $R_2$ et/ou $R_3$ comportent une ou plusieurs fonctions amine, hydroxyle ou ester d'acide carboxylique.

6. Procédé selon l'une des revendications 1 à 5, caractérisé en ce que l'on utilise des amines secondaires de formule générale (II)

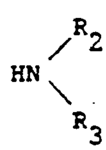 (II)

dans laquelle au moins un des symboles $R_2$ et $R_3$ et de préférence les deux symboles $R_2$ et $R_3$ représentent :

• un radical alkyle secondaire ayant de 3 à 10 atomes de carbone tel qu'isopropyle, butyle-2, pentyle-2, pentyle-3, hexyle-2, hexyle-3, heptyle-2, heptyle-3, heptyle-4, octyle-2, octyle-3, octyle-4, nonyle-2, nonyle-3, nonyle-4, nonyle-5, décyle-2, décyle-3, décyle-4 et décyle-5 ;

• un radical cyclohexyle ou cyclopentyle ;

ou des amines secondaires de formule générale (II) dans laquelle $R_2$ et $R_3$ forment avec l'atome d'azote un hétérocycle comportant éventuellement un autre atome d'azote ou un atome d'oxygène.

7. Procédé selon l'une des revendications 1 à 6, caractérisé en ce que l'on utilise comme amine la diisopropylamine, la diisobutylamine, la dibutylamine, la dicyclohexylamine, la morpholine, l'imidazole.

8. Procédé selon l'une des revendications 1 à 6, caractérisé en ce que l'on utilise comme amine la n-propylamine, l'isopropylamine, la n-butylamine, l'isobutylamine, la tertiobutylamine, la n-pentylamine, la méthyl-2 butylamine, la méthyl-3 butylamine, la n-hexylamine, la méthyle-2 pentylamine, la méthyl-3 pentylamine, l'éthyl-2 hexylamine, la laurylamine, la cyclohexylamine, la cyclopentylamine, la benzylamine, l'ester éthylique de la glycine ou l'éthanolamine.

9. Procédé selon l'une des revendications 1 à 8, caractérisé en ce que l'orthochlorophénol à chlorer se trouve en mélange avec essentiellement un ou plusieurs chlorophénols choisis parmi le dichloro-2,6 phénol,

le dichloro-2,4 phénol et le trichloro-2,4,6 phénol.

10. Procédé selon l'une des revendications 1 à 9, caractérisé en ce qu'il est mis en oeuvre à une température comprise entre le point de fusion de l'orthochlorophénol et des autres chlorophénols éventuellement présents et 150°C, et de préférence comprise entre 65°C et 120°C.

11. Procédé selon l'une des revendications 1 à 10, caractérisé :
– en ce que l'on procède tout d'abord à la chloration de l'orthochlorophénol à l'état fondu, en présence de 0,005% à 10% en poids d'une amine primaire, secondaire ou tertiaire jusqu'à obtention d'une concentration en orthochlorophénol dans le milieu réactionnel inférieure ou égale à environ 30% en poids,
– puis en ce que l'on poursuit la chloration en introduisant concomitamment le chlore et de l'orthochlorophénol de manière à maintenir la concentration pondérale dans le milieu réactionnel à une valeur inférieure ou égale à 30%.

12. Procédé de chloration du dichloro-2,6 phénol obtenu selon l'une des revendications 1 à 11, caractérisé en ce que l'on opère en présence d'une quantité efficace d'un acide fort ou d'un acide de Lewis.

13. Procédé selon la revendication 12, caractérisé en ce que l'acide fort est un acide protonique ayant une fonction d'acidité Ho inférieure ou égale à –5.

14. Procédé selon l'une des revendications 12 ou 13, caractérisé en ce que l'acide fort est choisi dans le groupe comprenant l'acide sulfurique, l'acide perchlorique, l'acide trifluorométhanesulfonique, l'acide chlorosulfonique, l'acide fluorosulfonique, l'acide pyrosulfurique et des résines acides comportant des groupements fluorosulfoniques.

15. Procédé selon la revendication 12, caractérisé en ce que l'acide de Lewis est choisi dans le groupe comprenant le chlorure d'aluminium, le bromure d'aluminium, les chlorures stannique et stanneux, les bromures stannique et stanneux, le trichlorure de bismuth, le tétrachlorure de titane, le tétrachlorure de zirconium, le pentafluorure d'antimoine, l'hexachlorure de tungstène, les chlorures de molybdène, le chlorure ferrique, le chlorure ferreux, le bromure ferrique, le bromure ferreux, le chlorure cuivreux, le chlorure cuivrique et le chlorure de zinc.

16. Procédé selon l'une des revendications 12 ou 15, caractérisé en ce que l'acide de Lewis est choisi parmi le chlorure d'aluminium, le chlorure ferrique, le tétrachlorure de zirconium et le tétrachlorure de titane.

17. Procédé selon l'une des revendications 12 à 16, caractérisé en ce que le rapport pondéral acide fort/dichloro-2,6 phénol ou acide de Lewis/dichloro-2,6 phénol est de 0,01% à 10% et de préférence de 0,1% à 5%.

**Claims**

1. Process for the chlorination of ortho-chlorophenol with gaseous chlorine to 2,6-dichlorophenol, characterized in that the reaction is performed in the molten state and in the presence of an effective quantity of at least one primary, secondary or tertiary amine.

2. Process according to claim 1 for the chlorination of ortho-chlorophenol with gaseous chlorine to 2,6-dichlorophenol, characterized in that the reaction is performed in the molten state in the presence of :
– at least one polychlorophenol in such quantity that the ratio by weight ortho-chlorophenol/total chlorophenols is less than or equal to 30%,
– and an effective quantity of at least one primary, secondary or tertiary amine.

3. Process according to one of claims 1 and 2, characterized in that the amine used is an amine of general formula (I) :

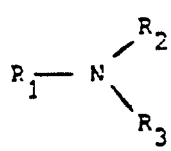

(I)

in which : $R_1$, $R_2$ and $R_3$, which may be identical or different, denote :
- a linear alkyl radical having from 1 to 12 carbon atoms, a secondary alkyl radical having 3 to 12 carbon atoms or a tertiary alkyl radical having 4 to 12 carbon atoms, it being possible for these alkyl radicals to contain one or two –O– ether groups or hydroxyl, amine, carboxylic acid, carboxylic acid ester, amide or imine groups ;
- a phenyl radical, a cyclohexyl radical, a cycloheptyl radical or a cyclopentyl radical ;
- a phenylalkyl, cyclohexylalkyl, cycloheptylalkyl or cyclopentylalkyl radical in which the alkyl portion contains 1 to 4 carbon atoms ;

- a hydrogen atom ;

$R_1$ can denote an $NH_2$ group ;

$R_2$ and $R_3$ form, together and with the nitrogen atom, a saturated heterocycle or a heterocycle containing one or more double bonds, optionally substituted with one or more alkyl groups having 1 to 4 carbon atoms ;

$R_2$ and $R_3$ or $R_1$, $R_2$ and $R_3$ form, together with the nitrogen atom and with one or more nitrogen and/or oxygen and/or sulphur atoms, a saturated or unsaturated heterocycle optionally substituted with one or more alkyl groups having 1 to 4 carbon atoms ;

$R_1$, $R_2$ and $R_3$ form, together and with the nitrogen atom, an unsaturated heterocycle optionally substituted with one or two methyl or ethyl groups ;

$R_2$ and $R_3$ or $R_1$, $R_2$ and $R_3$ form, together with the nitrogen atom and optionally with one or more nitrogen and/or oxygen and/or sulphur atoms, a saturated or unsaturated polycyclic compound optionally substituted with one or more alkyl groups having 1 to 4 carbon atoms.

4. Process according to one of claims 1 to 3, characterized in that the quantity of amine used represents from 0.005% to 10%, and preferably from 0.015% to 5%, by weight relative to the weight of the reaction medium.

5. Process according to one of claims 1 to 4, characterized in that use is made of primary or secondary amines of general formula (II) :

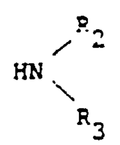

(II)

in which :

$R_2$ or $R_3$ denotes a hydrogen atom ;

$R_2$ and $R_3$, which may be identical or different, denote :

- a linear alkyl radical having 1 to 10 carbon atoms ;
- a secondary alkyl radical having 3 to 10 carbon atoms ;
- a tertiary alkyl radical having 4 to 10 carbon atoms ;
- a cyclohexyl or cyclopentyl radical ;
- a phenyl radical ;
- a benzyl or phenethyl radical ;

$R_2$ and $R_3$ form, together with the nitrogen atom and with another nitrogen and/or oxygen atom, a saturated heterocycle or a heterocycle containing one or more unsaturated bonds ;

$R_2$ and/or $R_3$ contain one or more amine, hydroxyl or carboxylic acid ester groups.

6. Process according to one of claims 1 to 5, characterized in that use is made of secondary amines of general formula (II) :

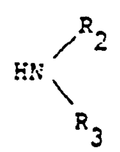

(II)

in which at least one of the symbols $R_2$ and $R_3$, and preferably both symbols $R_2$ and $R_3$, denote :

- a secondary alkyl radical having from 3 to 10 carbon atoms, such as isopropyl, 2-butyl, 2-pentyl, 3-pentyl, 2-hexyl, 3-hexyl, 2-heptyl, 3-heptyl, 4-heptyl, 2-octyl,–3-octyl, 4-octyl, 2-nonyl, 3-nonyl, 4-nonyl, 5-nonyl, 2-decyl, 3-decyl, 4-decyl and 5-decyl ;
- a cyclohexyl or cyclopentyl radical ; or secondary amines of general formula (II) in which $R_2$ and $R_3$ form, with the nitrogen atom, a heterocycle optionally containing another nitrogen atom or an oxygen atom.

7. Process according to one of claims 1 to 6, characterized in that diisopropylamine, diisobutylamine, dibutylamine, dicyclohexylamine, morpholine or imidazole is used as an amine.

8. Process according to one of claims 1 to 6, characterized in that n-propylamine, isopropylamine, n-butylamine, isobutylamine, tert-butylamine, n-pentylamine, 2-methylbutylamine, 3-methylbutylamine, n-hexylamine, 2-methylpentylamine, 3-methylpentylamine, 2-ethylhexylamine, laurylamine, cyclohexylamine, cyclopentylamine, benzylamine, glycine ethyl ester or ethanolamine is used as an amine.

9. Process according to one of claims 1 to 8, characterized in that the ortho-chlorophenol to be chlorinated

is in a mixture with, chiefly, one or more chlorophenols chosen from 2,6-dichlorophenol, 2,4-dichlorophenol and 2,4,6-trichlorophenol.

10. Process according to one of claims 1 to 9, characterized in that it is carried out at a temperature between the melting point of ortho-chlorophenol and of the other chlorophenols which may be present and 150°C, and preferably between 65°C and 120°C.

11. Process according to one of claims 1 to 10, characterized :

– in that the chlorination of the ortho-chlorophenol is performed in the first place in the molten state, in the presence of 0.005% to 10% by weight of a primary, secondary or tertiary amine, until a concentration of ortho-chlorophenol in the reaction medium of less than or equal to approximately 30% by weight is obtained,

– and then in that the chlorination is continued by simultaneously introducing chlorine and ortho-chlorophenol so as to maintain the concentration by weight in the reaction medium at a value of less than or equal to 30%.

12. Process for the chlorination of 2, 6-dichlorophenol obtained according to one of claims 1 to 11, characterized in that the reaction is performed in the presence of an effective quantity of a strong acid or Lewis acid.

13. Process according to claim 12, characterized in that the strong acid is a protonic acid having an acidity function Ho of less than or equal to –5.

14. Process according to one of claims 12 and 13, characterized in that the strong acid is chosen from the group comprising sulphuric acid, perchloric acid, trifluoromethanesulphonic acid, chlorosulphonic acid, fluorosulphonic acid, fuming sulphuric acid and acidic resins containing fluorosulphonic groups.

15. Process according to claim 12, characterized in that the Lewis acid is chosen from the group comprising aluminium chloride, aluminium bromide, stannic and stannous chlorides, stannic and stannous bromides, bismuth trichloride, titanium tetrachloride, zirconium tetrachloride, antimony pentafluoride, tungsten hexachloride, the molybdenum chlorides, ferric chloride, ferrous chloride, ferric bromide, ferrous bromide, cuprous chloride, cupric chloride and zinc chloride.

16. Process according to one of claims 12 and 15, characterized in that the Lewis acid is chosen from aluminium chloride, ferric chloride, zirconium tetrachloride and titanium tetrachloride.

17. Process according to one of claims 12 to 16, characterized in that the ratio by weight strong acid/2,6-dichlorophenol or Lewis acid/2,6-dichlorophenol is from 0.01% to 10%, and preferably from 0.1% to 5%.

## Ansprüche

1. Verfahren zur Chlorierung von o-Chlorphenol zu 2,6-Dichlorphenol durch Chlorgas, dadurch gekennzeichnet, daß man in der Schmelze und in Gegenwart einer wirksamen Menge wenigstens eines primären, sekundären oder tertiären Amins arbeitet.

2. Verfahren nach Anspruch 1 zur Chlorierung von o-Chlorphenol zu 2,6-Dichlorphenol durch Chlorgas, dadurch gekennzeichnet, daß man in der Schmelze in Gegenwart

– wenigstens eines Polychlorphenols in einer solchen Menge, daß das Gewichtsverhältnis o-Chlorphenol/ Gesamtheit der Chlorphenole kleiner oder gleich 30% ist und

– in Gegenwart einer wirksamen Menge wenigstens eines primären, sekundären oder tertiären Amins arbeitet.

– 3. Verfahren nach Anspruch 1 oder 2, dadurch gekennzeichnet, daß das verwendete Amin ein Amin der allgemeinen Formel (I)

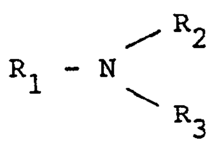

(I)

ist, worin

– $R_1$, $R_2$ und $R_3$ gleich oder verschieden sind und

– einen 1 bis 12 Kohlenstoffatome enthaltenden geradkettigen Alkylrest, einen 3 bis 12 Kohlenstoffatome enthaltenden sekundären Alkylrest oder einen 4 bis 12 Kohlenstoffatome enthaltenden tertiären Alkylrest, wobei diese Alkylreste eine oder zwei Ether-, –O–, oder Hydroxyl-, Amin-, Carbonsäure-, Carbonsäureester-, Amid- oder Iminfunktion(en) enthalten können,

– einen Phenyl-, Cyclohexyl-, Cycloheptyl- oder Cyclopentylrest,

– einen Phenylalkyl-, Cyclohexylalkyl-, Cycloheptylalkyl- oder Cyclopentylalkylrest, dessen Alkylteil 1 bis 4 Kohlenstoffatome enthält und
– ein Wasserstoffatom
bedeuten,
– $R_1$ eine $NH_2$-Gruppe bedeuten kann,
– $R_2$ und $R_3$ gemeinsam und mit dem Stickstoffatom einen, gegebenenfalls durch eine oder mehrere 1 bis 4 Kohlenstoffatome enthaltende(n) Alkylgruppe(n) substituierten, gesättigten oder eine oder mehrere Doppelbindung(en) enthaltenden Heterocyclus bilden,
– $R_2$ und $R_3$ oder $R_1$, $R_2$ und $R_3$ gemeinsam mit dem Stickstoffatom und mit einem oder mehreren Stickstoff- und/oder Sauerstoff- und/oder Schwefelatom(en) einen gegebenenfalls durch eine oder mehrere 1 bis 4 Kohlenstoffatome enthaltende(n) Alkylgruppe(n) substituierten gesättigten oder ungesättigten Heterocyclus bilden,
– $R_1$, $R_2$ und $R_3$ gemeinsam und mit dem Stickstoffatom einen gegebenenfalls durch eine oder zwei Methyl- oder Ethylgruppe(n) substituierten ungesättigten Heterocyclus bilden und
– $R_2$ und $R_3$ oder $R_1$, $R_2$ und $R_3$ gemeinsam mit dem Stickstoffatom und gegebenenfalls mit einem oder mehreren Stickstoff- und/oder Sauerstoff und/oder Schwefelatom(en) eine gegebenenfalls durch eine oder mehrere 1 bis 4 Kohlenstoffatome enthaltende Alkylgruppe(n) substituierte, gesättigte oder uncgesättigte polycyclische Verbindung bilden.

4. Verfahren nach einem der Ansprüche 1 bis 3, dadurch gekennzeichnet, daß die Menge des verwendenen Amins 0,005 bis 10 Gewichtsprozent und vorzugsweise 0,015 bis 5 Gewichtsprozent des Reaktionsmediums ausmacht.

5. Verfahren nach einem der Ansprüche 1 bis 4, dadurch gekennzeichnet, daß man primäre oder sekundäre Amine der allgemeinen Formel (II)

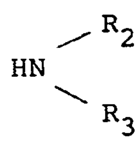

(II)

verwendet, worin
– $R_2$ oder $R_3$ ein Wasserstoffatom bedeutet,
– $R_2$ und $R_3$ gleich oder verschieden sind und
– einen 1 bis 10 Kohlenstoffatome enthaltenden geradkettigen Alkyl-,
– einen 3 bis 10 Kohlenstoffatome enthaltenden sekundären Alkyl-,
– einen 4 bis 10 Kohlenstoffatome enthaltenden gertiären Alkyl-,
– einen Cyclohexyl- oder Cyclopentyl-,
– einen Phenyl- und
– einen Benzyl- oder Phenethylrest bedeuten,
– $R_2$ und $R_3$ gemeinsam mit dem Stickstoffatom und mit einem anderen Stickstoff- und/oder Sauerstoffatom einen gesättigten oder eine oder mehrere ungesättigte Bindung(en) enthaltenden Heterocyclus bilden und
– $R_2$ und/oder $R_3$ eine oder mehrere Amin-, Hydroxyl- oder Carbonsäureesterfunktion(en) enthalten.

6. Verfahren nach einem der Ansprüche 1 bis 5, dadurch gekennzeichnet, daß man sekundäre Amine der allgemeinen Formel (II) verwendet,

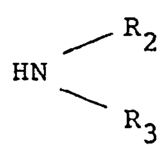

(II)

worin wenigstens eines der Symbole $R_2$ und $R_3$ und vorzugsweise beide Symbole $R_2$ und $R_3$
– einen 3 bis 10 Kohlenstoffatome enthaltenden sekundären Alkylrest wie Isopropyl, 2-Butyl, 2-Pentyl, 3-Pentyl, 2-Hexyl, 3-Hexyl, 2-Heptyl, 3-Heptyl, 4-Heptyl, 2-Octyl, 3-Octyl, 4-Octyl, 2-Nonyl, 3-Nonyl, 4-Nonyl, 5-Nonyl, 2-Decyl, 3-Decyl, 4-Decyl, 5-Decyl und
– einen Cyclohexyl- oder Cyclopentylrest
oder sekundäre Amine der allgemeinen Formel (II) bedeuten, in der $R_2$ und $R_3$ mit dem Stickstoffatom einen gegebenenfalls ein anderes Stickstoff- oder Sauerstoffatom enthaltenden Heterocyclus bilden.

7. Verfahren nach einem der Ansprüche 1 bis 6, dadurch gekennzeichnet, daß man als Amin, Diisopropylamin, Diisobutylamin, Dibutylamin, Dicyclohexylamin, Morpholin und Imidazol verwendet.

8. Verfahren nach einem der Ansprüche 1 bis 6, dadurch gekennzeichnet, daß man als Amin n-Propylamin, Isopropylamin, n-Butylamin, Isobutylamin, tert.-Butylamin, n-Pentylamin, 2-Methylbutylamin, 3-Methylbutylamin, n-Hexylamin, 2-Methylpentylamin, 3-Methylpentylamin, 2-Ethylhexylamin, Laurylamin, Cyclohexylamin, Cyclopentylamin, Bernzylamin, Aminoessigsäureethylester oder Ethanolamin verwendet.

9. Verfahren nach einem der Ansprüche 1 bis 8, dadurch gekennzeichnet, daß das zu chlorierende o-Chlorphenol hauptsächlich mit einem oder mehreren Chlorphenolen gemischt vorliegt, die aus 2,6-Dichlorphenol, 2,4-Dichlorphenol und 2,4,6-Trichlorphenol ausgewählt sind.

10. Verfahren nach einem der Ansprüche 1 bis 9, dadurch gekennzeichnet, daß es bei einer Temperatur zwischen dem Schmelzpunkt des o-Chlorphenols und dem der anderen gegebenenfalls vorhandenen Chlorphenole und 150°C, vorzugsweise zwischen 65°C und 120°C durchgeführt wird.

11. Verfahren nach einem der Ansprüche 1 bis 10, dadurch gekennzeichnet

– daß man zunächst die Chlorierung des o-Chlorphenols in der Schmelze in Gegenwart von 0,005 bis 10 Gewichtsprozent primärem, sekundärem oder tertiärem Amin bis zum Erhalten einer Konzentration des o-Chlorphenols von kleiner oder gleich etwa 30 Gewichtsprozent des Reaktionsmediums durchführt und

– daß man anschließend die Chlorierung unter gemeinsamer Chlor- und o-Chlorphenolzugabe derart fortsetzt, daß die Konzentration bei einem Wert kleiner oder gleich 30 Gewichtsprozent des Reaktionsmediums erhalten bleibt.

12. Verfahren zur Chlorierung zu 2,6-Dichlorphenol, erhalten nach einem der Ansprüche 1 bis 11,dadurch gekennzeichnet, daß man in Gegenwart einer wirksamen Menge einer starken Säure oder einer Lewis- Säure arbeitet.

13. Verfahren nach Anspruch 12, dadurch gekennzeichnet, daß die starke Säure eine Protonensäure mit einem Säureexponenten $pK_S$ von kleiner oder gleich –5 ist.

14. Verfahren nach Anspruch 12 oder 13, dadurch gekennzeichnet, daß die starke Säure aus der Gruppe ausgewählt ist, welche Schwefel-, Perchlor-, Trifluormethansulfon-, Chlorsulfon-, Fluorsulfon-, Dischwefelsäure und Fluorsulfonsäuregruppen enthaltende Säureharze umfaßt.

15. Verfahren nach Anspruch 12, dadurch gekennzeichnet, daß die Lewis-Säure aus der Gruppe ausgewählt ist, welche Aluminiumchlorid, Aluminiumbromid, Zinn(II)- und Zinn(IV)-chlorid, Zinn(II)- und Zinn(IV)-bromid, Bismuttrichlorid, Titantetrachlorid, Zirkoniumtetrachlorid, Antimonpentafluorid, Wolframhexachlorid, die Molybdänchloride, Eisen(II)- und Eisen(III)-chlorid, Eisen(II)- und Eisen(III)-bromid, Kupfer(I)- und Kupfer(II)- chlorid und Zinkchlorid umfaßt.

16. Verfahren nach Anspruch 12 oder 15, dadurch gekennzeichnet, daß die Lewis-Säure aus Aluminiumchlorid, Eisen(III)-chlorid, Zirkoniumtetrachlorid und Titantetrachlorid ausgewählt ist.

17. Verfahren nach einem der Ansprüche 12 bis 16, dadurch gekennzeichnet, daß das Gewichtsverhältnis von starker Säure/2,6-Dichlorphenol oder Lewis-Säure/ 2,6-Dichlorphenol 0,01 bis 10% und vorzugsweise 0,1 bis 5% beträgt.